Europäisches Patentamt

European Patent Office

Office européen des brevets

(11) Publication number: **0 442 194 A2**

# EUROPEAN PATENT APPLICATION

(21) Application number: 90311214.2

(51) Int. Cl.5: **A61M 25/01**

(22) Date of filing: **12.10.90**

(30) Priority: **13.02.90 US 479387**

(43) Date of publication of application:
**21.08.91 Bulletin 91/34**

(84) Designated Contracting States:
**DE ES FR GB IT**

(71) Applicant: **DAIG CORPORATION**
**14901 Minnetonka Industrial Road**
**Minnetonka, MN 55345(US)**

(72) Inventor: **Fleischhacker, John J.**
**16631 Meadowbrook Lane**
**Wayzata, MN 55391(US)**
Inventor: **Hidani, Tim T.**
**886 Tatum Street**
**St. Paul, MN 55104(US)**
Inventor: **Krueger, Mark D.**
**8249 West River Road**
**Minneapolis, MN 55444(US)**

(74) Representative: **De Minvielle-Devaux, Ian**
**Benedict Peter et al**
**CARPMAELS & RANSFORD 43, Bloomsbury**
**Square**
**London WC1A 2RA(GB)**

(54) **Cylindrical plug for sealing around guidewire.**

(57) Disclosed herein is a catheter introducer comprising a longitudinally extended body having disposed therein a cylindrical hemostasis plug. The plug has a first face which contains a pilot opening which partially penetrates the plug body and a second face which contains a slit which partially penetrates the plug body from the opposite direction. In the preferred embodiment the slit and the pilot opening are aligned and permit, thereby, the use of the introducer set with a wide variety of catheter and guidewire diameters while at the same time foreclosing the possibility of the formation of clots or of the leakage of blood through the hemostasis plug.

Fig. 1

EP 0 442 194 A2

## CYLINDRICAL PLUG FOR SEALING AROUND GUIDEWIRE

Cross Reference to Related Applications
NONE

Statement as to Rights to Inventions Made Under Federally Sponsored Research and Development
NONE

Background of Invention

1. Field of Invention

This invention relates to medical instruments. More particularly, this invention relates to hemostasis plugs, or the like which may be used in a wide variety of medical procedures.

2. Prior Art

The introduction of catheters into blood vessels for a variety of purposes such as coronary angiography has been known for many years. Several techniques for introducing these catheters are available. One such technique is the cut-down method. Another is the Seldinger technique. This technique involves surgically opening a vein or artery with a needle, inserting a guidewire into the vein or artery through the lumen of the needle, withdrawing the needle, inserting over the guidewire a dilator located inside an associated hemostasis valve and sheath, removing the dilator and inserting a catheter through the hemostasis valve and sheath and into the blood vessel.

A wide variety of hemostasis valves are known in the prior art. However, each of these prior art devices suffers from at least one of the following defects:

a. When a guidewire is inserted through the body containing a hemostasis valve, because the guidewire is so small relative to other catheters which may also be employed, it is difficult for the valve to seal against the backward pressure of blood, while at the same time permitting easy insertion of much larger diameter catheters. This problem is particularly acute with the procedures involving arterial invasion where there is a high reverse pressure of blood. In these arterial procedures, blood can often squirt out when the guidewire is first introduced through the hemostasis valve. Excessive blood leakage is viewed by many as extremely dangerous.

b. If a proper internal structure is not maintained within the body containing the hemostasis valve, clots are likely to form. These clots, in turn, may be returned to the blood vessels during catheter placement and/or exchanges causing undesirable medical consequences for the patient.

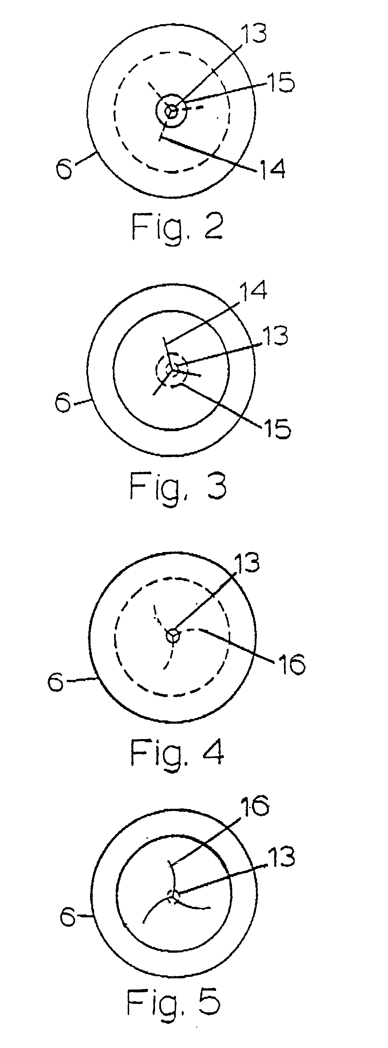

U.S. Patent No. 4,000,739 suffers from both of these deficiencies. Specifically, the hole (26), is described as being similar in size to the body of the cannula (46) which is introduced into the body. Since, as shown in Fig. 3, the guidewire is several orders of magnitude smaller than the cannula body, any guidewire inserted through the valve is not sealed by the disc (22). While some sealing does occur with the disc (24), the sealing is often not sufficient to stop a reverse flow of blood when only the guidewire is present. Because the discs (22) and (24) function independently, it is often possible for the guidewire to move off center of the disc (24), thereby causing a further leakage of blood. In addition, independent movement between discs (22) and (24) permits the formation of a small recess around the cannula body where it meets the disc (22). As the cannula is manipulated, blood can be forced into the interstices between (22) and (24). When a new catheter is inserted, the clots which have formed in this area may be inadvertently introduced into the patient's blood stream.

The device described in U.S. Patent No. 4,673,393, particularly in Fig. 4, suffers from similar deficiencies in that blood build-up may occur in the small area at the point where the discs (16) and (17) join on the cannula as shown in Fig. 3. In addition, the devices described in Figs. 4 and 5 do not force the centering of the guidewire. Instead, the guidewire may move off-center of the slits and thereby increase the likelihood of a backward flow of blood. A similar problem is seen with the plug described in U.S. Patent No. 4,610,655 in Figs 4 and 5(a).

U.S. Patent No. 4,655,752 discloses a surgical cannula which again suffers from the deficiency that the first sealing member (79) will not center a guidewire. In addition, an area between the first and second sealing members is provided in which blood can build up and ultimately form clots.

U.S. Patent No. 4,634,432 describes an introducer sheath assembly which contains a single disc (52) having a single slit therein. This disc suffers from the same deficiency that prior slit discs suffer from in that they permit movement of a guidewire to an off-center position which in turn will permit reverse leakage of blood.

U.S. Patent No. 4,424,833 discloses a self-sealing gasket assembly which contains a space between the seals (42) and (44). Within this space, blood may accumulate, particularly when a cannula is withdrawn. In addition, as the cannula is inserted, the disc (44) is caused to expand along the cannula wall, thus increasing the size of the space (53) and providing a greater area for the accumulation of undesirable clots. Finally, the opening (48) is of sufficiently large size to permit a great deal of movement of a guidewire which in turn will permit increased leakage of blood. Because of the separate nature of the discs, the valve described in the '833 patent functions in a very similar fashion to the valve described in the '739 patent and thereby possesses both the deficiencies described above.

U.S. Patent No. 4,798,594 discloses a hemostasis valve comprised of a plurality of helical slits. Because a pilot or other guiding hole is not present, the guidewire introduced into this valve can readily be skewed off-center, thereby permitting the reverse flow of blood. In a similar fashion, U.S. Patent No. 4,626,245 describes a valve body containing two Y-shaped slits which are offset one from the other. This offset alignment permits a guidewire inserted into the cannula to move into an off-center position and thereby encourages the reverse leakage of blood.

U.S. Patent No. 4,430,081 discloses the use of three essentially flat discs, one containing a slit, one containing a hole, and one containing a C-shaped cut. As a catheter is inserted into the discs, each of the discs is displaced in the direction of insertion, thereby creating undesirable spaces in which blood may accumulate and clots may form.

Accordingly, it is an object of this invention to prepare a hemostasis plug which can effectively seal around both guidewires and cannulas of varying diameters.

It is another object of this invention to prepare a hemostasis plug which does not encourage the retention of blood clots.

It is yet another object of this invention to prepare a cylindrical hemostasis plug which is held in the desired position and is not readily displaced by the action of a cannula inserted therethrough.

It is a further object of this invention to prepare a hemostasis plug which will effectively center guidewires to ensure that they do not move to an off-center position, thereby permitting leakage of blood. These and other objectives are obtained by utilizing the device described hereafter.

## Summary of Invention

Described herein is a cylindrical hemostasis plug for use in conjunction with a catheter introducer set. The plug has first and second parallel, generally circular faces. The first face is provided with a pilot opening having a diameter of less than that of the guidewire. The opening partially penetrates the body of the plug in a direction generally perpendicular to the first face. The plug also has a second face which is provided with a slit or slits generally aligned with the pilot opening. The slit itself is perpendicular to the second face and partially penetrates the plug body. Thus, the resulting hemostasis plug effectively seals around both catheters and guidewires. Moreover, the pilot opening forces the guidewire to remain in a centered position within the plug, thereby controlling the leakage of blood which often occurs when the guidewire slips to an off-center position. In addition, this hemostasis plug, because of its solid, one-piece nature, does not permit the creation of undesirable blood clots.

## Brief Description of Drawings

1. Figure 1 is a cross-sectional view of the hemostasis plug of the instant invention in place within a hemostasis body/cannula assembly.
2. Figure 2 is a top view of the hemostasis plug of Fig. 1.
3. Figure 3 is a bottom view of the hemostasis plug of Fig. 1.
4. Figure 4 is a top view of another version of the hemostasis plug of the instant invention.
5. Figure 5 is a bottom view of the Fig. 4 hemostasis plug.

## Detailed Description of Invention

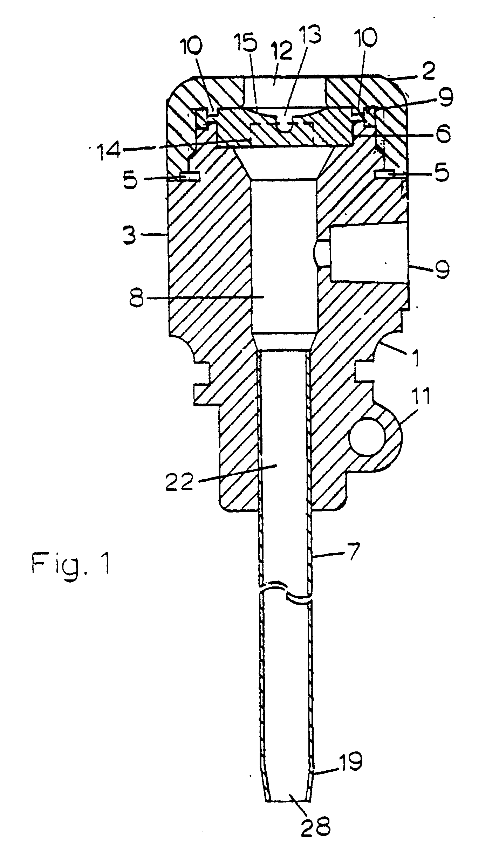

Turning first to Figure 1 which shows a cross-sectional view of the hemostasis cannula unit of this invention, the cannula unit (1) is formed from four major parts. The first is the cap means (2) which is attached to the top of the longitudinally extended, valve housing (3) and is provided with a first opening (12) which cooperates with a second opposing opening (22) located in the valve housing (3) to permit insertion of a catheter into and out of the interior of the valve housing (3). The cap means (2) and the valve housing (3) are formed from a relatively hard plastic such as a polycarbonate or a polyurethane. The cap means (2) may be secured to the valve housing (3) by mechanically attaching to the housing using threads, clips or, as shown in the drawings, by snap fittings, gluing or welding. The third major element of the hemostasis cannula unit of the instant invention, the hemostasis plug (6), is formed from a pliant, resilient rubber such as silicone rubber or latex rubber, which can be shaped to readily admit passage of catheters. The final major element of the hemostasis cannula unit of the instant invention is a tubular sheath (7) which is formed from a relatively rigid plastic such as teflon or polyethylene. The sheath is inserted within the valve housing (3) and cooperates to provide an exit from the interior of the valve housing (8).

As shown in Figure 1, the hemostasis plug (6), the cap means (2) and the valve housing (3) are joined together by inserting the plug (6) into the valve housing (3) such that the uppermost edge of the plug is in contact with the rib (10), which is preferably circular in nature. The cap means (2) is then attached to the valve housing (3). The cap means may be attached to the valve housing by snap fittings or by the use of suitable adhesives, or by otherwise causing the plastic in the cap to melt onto the valve housing. The circular groove (5) provides a space for the plastic material from the cap means (2) and the housing (3) to flow into as these materials flow together during the attachment process.

The cap means (2) is provided with a first opening (12) at the top, and may also be provided with a Luer taper (not shown) which can receive corresponding male Luer taper fittings (not shown).

The plug (6) has a pilot opening (13) which leads into the center of a slit (14) which is preferably Y-shaped, as shown in Figure 3, but may also be bent Y-shaped, as shown in Figure 5. In addition, the slit (14) may be in the form of a cross or a single slit. Preferably, the pilot hole leads directly to the center of the Y. The diameter of the pilot opening (13) is slightly smaller than the diameter of a guidewire (not shown) which will be used with the hemostasis cannula unit.

The hemostasis plug (6) is provided with a target receiving area (15) to aid in inserting catheters and guidewires. If the catheter is inserted slightly off-center, it will be readily guided into the pilot opening (13) and through the slits (14).

The valve housing itself (3) is generally longitudinally extended to form a valve chamber (8) and having a second opening (22) opposing the first opening (12) in the cap means (2). This arrangement allows a catheter to be inserted through the cap means (2), and into and out of the valve chamber (8). preferably access to the interior of the chamber is also provided through a port (9) which facilitates insertion or withdrawal of fluids from the chamber (8).

The valve housing (3) of the hemostasis cannula unit (1) is also provided with a suture loop (11) to allow temporary attachment of the cannula unit directly to a patient's body to provide stabilization of the hemostasis cannula unit.

The final element of the hemostasis cannula unit of the instant invention is the sheath (7) onto which the valve housing (3) may be attached. The sheath preferably is provided with a tapered distal tip (19), in the preferred use to closely fit onto a dilator which is inserted through the cannula for initial introduction into a blood vessel.

In the preferred means of operation, a needle is inserted into a patient's blood vessel. Through the lumen of the needle a guidewire is in turn inserted into the blood vessel. The hemostasis cannula unit of the instant invention is then prepared by inserting a dilator through the first opening (12), the plug (6), out the second opening (22), through the sheath (7), and out the distal end of the sheath (28). The sheath (7) and dilator are designed such that the tapered distal tip (19) snugly fits around the dilator.

Once the needle is removed leaving only the guidewire protruding from the patient, the dilator and hemostasis cannula unit are advanced as a single unit onto the guidewire and into the blood vessel. The dilator tip, which is tapered, increases the size of the opening in the blood vessel as it enters the vessel. Sheath (7) continues to increase the opening in the vessel as it is further inserted. After the sheath is inserted into the blood vessel, the dilator is removed, leaving in place the hemostasis cannula unit of the instant invention with the guidewire protruding therefrom, sealed by the pilot opening (13) and the slits (14).

A catheter may then be inserted through the first opening (12) in the cap means (2) and into the plug (6) through the pilot opening (13) and slits (14). After exiting the slits (14), the catheter is advanced out the second opening (22) down through the sheath (7) and into the blood vessel. Any blood which flows between the sheath and the catheter and up into the interior of the valve body (8) is not permitted to escape to the exterior because of the strong sealing action of the slits (14).

Figure 2 provides a top view of the plug of the instant invention. The pilot opening (13) is shown as are the slits (14) and the target receiving area (15).

Figure 3 is a bottom view of the plug shown in Figure 2 with the slits (14), the pilot opening (13) and the target receiving area (15) shown.

Figures 4 and 5 illustrate respectively top and bottom views of a hemostasis plug according to the instant invention in which a bent Y-shaped slit (16) is employed. The pilot opening (13) is also shown. The illustrated hemostasis plug is not provided with a target receiving area.

The hemostasis plug of the instant invention provides several advantages not found in prior art sealing valves. A major advantage of the plug of the instant invention is that because of its one-piece nature there is a significantly reduced possibility for clots to form within it. Many prior art hemostasis cannulas are constructed utilizing multiple valves placed in face-to-face or nearly face-to-face proximity. However, small amounts of blood which can form clots can easily accumulate between the valve faces. The plug of the instant invention, on the other hand, does not permit the accumulation of blood within its structure. Another major problem faced by hemostasis cannulas of the prior art is that they are unable to effectively seal around guidewires. Moreover, many of the prior art hemostasis valves permit guidewires to be displaced toward the edge of slitted valve bodies. The structure of the instant invention precludes displacement of this type. Because the guidewire is held in place by the pilot opening, it cannot readily be displaced to the side into one of the arms of the slits. Finally, the hemostasis plug of the instant invention finds particular importance because, unlike prior art hemostasis valves, it provides effective sealing action against high pressure arterial blood, even when only a guidewire is in place.

The present embodiment of the instant invention is considered to be merely illustrative and changes may be made in its specific form without departing from the spirit or essential characteristics of this invention.

## Claims

1. A cylindrical plug for use in conjunction with a catheter introducer set having first and second parallel, generally circular faces, said first face is provided with a pilot opening having a diameter of less than that of the guidewire which opening partially penetrates the body of the plug in a direction generally perpendicular to said first face and said second face having a slit aligned with said pilot opening, which slit is generally perpendicular to said second face and which partially penetrates the plug body.

2. The plug of Claim 1 wherein the pilot opening includes a target receiving area for receiving catheters to be inserted through the cylindrical plug.

3. The cylindrical plug of Claim 1 wherein the slit is Y-shaped.

4. The cylindrical plug of Claim 3 wherein at least one of the arms of the Y-shaped slit is bent.

5. The cylindrical plug of Claim 4 wherein all of the arms of the slit are bent.

6. The cylindrical plug of Claim 1 in combination with a longitudinally extended housing having an opening aligned with the pilot opening and an exit port aligned with the slit in said second face.

7. The cylindrical plug of Claim 6 wherein said exit port is attached to an extended cannula.

8. The cylindrical plug of Claim 7 in combination with a side port opening into the interior of the housing at a point between the plug and the exit port.

9. The cylindrical plug of Claim 1 wherein the pilot opening is aligned with the center of the slit.

10. A medical instrument for use in conjunction with a catheter comprising a longitudinally extended housing having an entry opening and an exit opening and having disposed in the interior thereof a cylindrical plug having first and second generally circular faces, said first face having a generally circular pilot opening having a diameter of less than said guidewire which partially penetrates the body of the plug in a direction perpendicular to said first face and said second face having a slit aligned with said pilot opening which slit is generally perpendicular to said second face and which partially penetrates the plug body.

11. A catheter introducer set for use in conjunction with a catheter comprising a longitudinally extended housing having an entry opening and an exit opening and having disposed in the interior thereof a cylindrical plug having first and second generally circular faces, said first face having a generally circular pilot opening having a diameter of less than said guidewire which partially penetrates the body of the plug in a direction perpendicular to said first face and said second face having a slit aligned with said pilot opening which slit is generally perpendicular to said second face and which partially penetrates the plug body.

Fig. 1

Fig. 2

Fig. 3

Fig. 4

Fig. 5